# EUROPÄISCHE PATENTSCHRIFT

(11) **EP 4 299 020 B1**
(45) Veröffentlichungstag und Bekanntmachung des Hinweises auf die Patenterteilung: **10.12.2025**
(21) Anmeldenummer: 23177257.5
(22) Anmeldetag: 05.06.2023
(51) Int. Cl.: A61B 17/29, A61B 90/00, A61B 17/28

(54) **CHIRURGISCHES INSTRUMENT**
SURGICAL INSTRUMENT
INSTRUMENT CHIRURGICAL

(30) Priorität: 30.06.2022 DE 102022116306
(43) Veröffentlichungstag der Anmeldung: 03.01.2024
(73) Patentinhaber: MED-EL Elektromedizinische Geräte GmbH, 6020 Innsbruck (AT)
(72) Erfinder: Kuen, Clemens, 6460 Imst (AT); Steinhardt, Uwe, 72145 Hirrlingen (DE); Schweissgut, Magdalena, 6067 Absam (AT)
(74) Vertreter: Witte, Weller & Partner Patentanwälte mbB

(56) Entgegenhaltungen:
- EP-A2- 0 543 107
- DE-U1- 202007 001 604
- DE-U1- 29 610 479
- US-A1- 2010 268 268

## Beschreibung

Die Erfindung betrifft ein chirurgisches Instrument mit den Merkmalen des Oberbegriffs des Anspruchs 1.

Das Gebrauchsmuster DE 20 2007 001 604 U1 offenbart eine laparoskopische Zange mit einem scherengriffartigen Handgriff, der zwei stabförmige Griffstücke aufweist, die zu einer Betätigung der Zange in Bezug zueinander beweglich, nämlich wie Hand- oder Fingerhebel einer Schere zueinander schwenkbar sind. Die Griffstücke weisen an ihren freien Enden Fingerringe auf, in denen austauschbare elastische Ringe zur Anpassung an individuelle Bedürfnisse eines Chirurgen angeordnet sind.

Die Patentanmeldung US 2010/0 268 268 A1 offenbart ein chirurgisches Instrument mit einem feststehenden Handgriff und mit einem Fingerring, der zu einer Betätigung des chirurgischen Instruments entlang einer geraden Schiebeführung zu dem Handgriff hin und von dem Handgriff weg verschiebbar ist. Die Schiebeführung ist mit einer Verriegelung gegen eine Verschiebung gesichert, die vorrübergehend mit einer Taste, die auf einer dem feststehenden Handgriff gegenüberliegenden Seite in dem Fingerring angeordnet ist, und dauerhaft mit einem um 90° umlegbaren Fingerhebel außerhalb des Fingerrings entriegelbar ist.

Die Patentanmeldung EP 0 543 107 A2 offenbart ein chirurgisches Instrument mit einem Scherengriff zu seiner Betätigung, der wie ein Pistolengriff seitlich von dem chirurgischen Instrument absteht, und mit einer Rasteinrichtung zu einer Feststellung des chirurgischen Instruments in einer betätigten Stellung verriegelbar, die mit einem nach Art eines Abzugs einer Pistole angeordneten Fingerhebel einrastbar ist.

Für verschiedene chirurgische Tätigkeiten ist es ein Vorteil, wenn eine Klemmkraft einer chirurgischen Zange bekannt oder begrenzt ist.

Aufgabe der Erfindung ist deswegen, ein chirurgisches Instrument vorzuschlagen, das eine Art Druckpunkt aufweist. Der Druckpunkt ist eine festgelegte oder festlegbare Betätigungskraft des chirurgischen Instruments. Das Erreichen der festgelegten oder festlegbaren Betätigungskraft des erfindungsgemäßen chirurgischen Instruments soll haptisch wahrnehmbar sein.

Das erfindungsgemäße chirurgische Instrument weist zwei beispielsweise stabförmige Griffstücke auf, die zu einer Betätigung des chirurgischen Instruments gegeneinander beweglich sind. Insbesondere ist zur Betätigung des chirurgischen Instruments ein Abstand der Griffstücke voneinander verkleinerbar. Es können beide Griffstücke beweglich sein oder es ist ein Griffstück feststehend und das andere Griffstück beweglich.

Eines der beiden Griffstücke, das zu seiner eindeutigen Bezeichnung und zur Unterscheidung vom anderen Griffstück hier nachfolgend als zweites Griffstück bezeichnet werden wird, weist zwei Fingerauflagen auf einer dem anderen, hier nachfolgend als erstem bezeichneten Griffstück abgewandten Seite auf. Die Fingerauflagen sind Elemente, die zu einer Auflage eines oder auch mehrerer Finger einer das chirurgische Instrument an seinen Griffstücken haltenden Hand zur Betätigung des chirurgischen Instruments vorgesehen sind. Zur Betätigung des chirurgischen Instruments wird eine Betätigungskraft in Richtung des ersten Griffstücks auf eine erste der beiden Fingerauflagen ausgeübt. Die erste Fingerauflage überträgt die Betätigungskraft auf das zweite Griffstück derart, dass das zweite Griffstück mit der Betätigungskraft in Richtung des ersten Griffstücks beaufschlagt wird.

Die erste Fingerauflage ist in der Betätigungsrichtung elastisch, das heißt in Richtung des ersten Griffstücks beweglich auf der dem ersten Griffstück abgewandten Seite des zweiten Griffstücks angeordnet derart, dass sich die erste Fingerauflage an dem zweiten Griffstück in Richtung des ersten Griffstücks bewegt, wenn die Betätigungskraft in Richtung des ersten Griffstücks auf die erste Fingerauflage ausgeübt wird. Eine Bewegung, das heißt ein Weg oder eine Strecke, um die sich die erste Fingerauflage in Bezug auf das zweite Griffstück bewegt, wenn die Betätigungskraft auf die erste Fingerauflage ausgeübt wird, ist von einer Höhe der Betätigungskraft abhängig. Die Abhängigkeit zwischen Weg und Kraft kann proportional oder nicht proportional sein. Je größer die Betätigungskraft ist, die die erste Fingerauflage in der Betätigungsrichtung, das heißt in Richtung des ersten Griffstücks beaufschlagt, desto größer ist die Bewegung der ersten Fingerauflage am zweiten Griffstück beziehungsweise in Bezug auf das zweite Griffstück, an dem beide Fingerauflagen angeordnet sind.

Die zweite Fingerauflage ist vorzugsweise starr auf der dem ersten Griffstück abgewandten Seite des zweiten Griffstücks angeordnet. Die zweite Fingerauflage weist einen Versatz in Richtung des ersten Griffstücks, das heißt in der Betätigungsrichtung in Bezug zu der ersten Fingerauflage auf, wenn die erste Fingerauflage unbelastet ist, also nicht mit der Betätigungskraft beaufschlagt wird und infolge dessen unverformt ist. Durch die Beaufschlagung der ersten Fingerauflage mit der Betätigungskraft in der Betätigungsrichtung lässt sich die erste Fingerauflage am zweiten Griffstück in Bezug zu der zweiten Fingerauflage so weit bewegen, dass dem ersten Griffstück abgewandte Seiten oder Flächen der beiden Fingerauflage bündig sind. "Bündig" bedeutet, dass die dem ersten Griffstück abgewandte Seiten oder Flächen der beiden Fingerauflagen oder zumindest einander nahe beziehungsweise benachbarte Bereiche der dem ersten Griffstück abgewandten Seiten oder Flächen der beiden Fingerauflagen sich in einer Ebene oder auch in einer gemeinsamen, beispielsweise gekrümmten Fläche befinden beziehungsweise einen gleichen Abstand vom ersten Griffstück aufweisen. Die mit der zweiten Fingerauflage bündige Lage oder Position der ersten Fingerauflage entspricht dem Druckpunkt des erfindungsgemäßen chirurgischen Instruments. Die Erfindung schließt nicht aus, dass sich der erste Fingerauflage über die mit der zweiten Fingerauflage bündige Lage oder Position, das heißt über den Druckpunkt hinwegbewegen lässt.

In der Betätigungsrichtung gesehen ist die zweite Fingerauflage innerhalb der ersten Fingerauflage, beispielsweise in einer Öffnung oder einem Loch der ersten Fingerauflage, oder unmittelbar oder mit geringem Abstand neben oder in Verlängerung der ersten Fingerauflage auf der dem ersten Griffstück abgewandten Seite des zweiten Griffstücks angeordnet. Die zweite Fingerauflage ist in Bezug zu der ersten Fingerauflage so angeordnet, dass ein Finger einer das chirurgische Instrument an seinen beiden Griffstücken haltenden Hand, der auf die erste Fingerauflage gelegt wird und die Betätigungskraft auf die erste Fingerauflage ausübt, in Anlage an die zweite Fingerauflage gelangt, wenn der Finger die elastisch an dem zweiten Griffstück angeordnete erste Fingerauflage so weit in der Betätigungsrichtung bewegt hat, dass die erste Fingerauflage mit der zweiten Fingerauflage bündig ist. Dieses "in Anlage gelangen" des die erste Fingerauflage mit der Betätigungskraft in der Betätigungsrichtung beaufschlagenden Fingers an der zweiten Fingerauflage haptisch als "Druckpunkt" wahrgenommen. Dem Druckpunkt ist eine bestimmte (Höhe der) Betätigungskraft zugeordnet, nämlich die Betätigungskraft, die notwendig ist, um die erste Fingerauflage elastisch bis in die mit der zweiten Fingerauflage bündige Lage oder Position zu bewegen.

Ein Vorteil der Erfindung ist, dass der Druckpunkt bei der Betätigung des chirurgischen Instruments haptisch wahrgenommen wird: der Finger, der zur Betätigung des chirurgischen Instruments die erste Fingerauflage am zweiten Griffstück des chirurgischen Instruments mit einer Betätigungskraft in der Betätigungsrichtung beaufschlagt, spürt, wenn die ursprünglich entgegen der Betätigungsrichtung zur zweiten Fingerauflage versetzte erste Fingerauflage in die mit der zweiten Fingerauflage bündige Lage oder Position gelangt. Ein Chirurg oder allgemein eine das erfindungsgemäße chirurgische Instrument benutzende Person erhält dadurch eine Rückmeldung über das Erreichen der Betätigungskraft, die der bündigen Lage der beiden Fingerauflagen, das heißt dem Druckpunkt zugeordnet ist. Das ermöglicht die Betätigung des chirurgischen Instruments mit der dem Druckpunkt zugeordneten Betätigungskraft und eine Begrenzung der Betätigungskraft auf die dem Druckpunkt zugeordnete Betätigungskraft.

Es wird darauf hingewiesen, dass die Bewegung der ersten Fingerauflage in Bezug auf das zweite Griffstück und in Bezug auf die zweite Fingerauflage eine andere Bewegung ist als die Bewegung, die sich die beiden Griffstücke bei der Betätigung des chirurgischen Instruments aufeinander zu bewegen. Die erste Fingerauflage bewegt sich mit dem zweiten Griffstück zusammen in Bezug zu dem ersten Griffstück und zusätzlich bewegt sich die erste Fingerauflage durch die Beaufschlagung mit der Betätigungskraft elastisch an beziehungsweise in Bezug auf das zweite Griffstück, an dem die erste Fingerauflage elastisch angeordnet ist. Die Bewegung der ersten Fingerauflage in Bezug auf das zweite Griffstück, an dem sie angeordnet ist, ist vorzugsweise kürzer und insbesondere nur ein Bruchteil der Bewegung des zweiten Griffstücks in Bezug zu dem ersten Griffstück. Der Versatz der beiden Fingerauflagen in der Betätigungsrichtung beträgt beispielsweise 1 mm oder weniger oder beispielsweise bis zu 2 mm oder bis zu 3 mm.

Das erfindungsgemäße chirurgische Instrument kann ein Federelement, beispielsweise eine Schraubendruckfeder, eine Schraubenzugfeder oder eine Blattfeder aufweisen, das die erste Fingerauflage in der Betätigungsrichtung elastisch beziehungsweise federnd am zweiten Griffstück hält.

Eine Ausgestaltung der Erfindung sieht eine mit dem zweiten Griffstück einstückige Ausbildung der ersten Fingerauflage vor, die - insbesondere durch ihre Form - in der Betätigungsrichtung elastisch an dem beziehungsweise in Bezug auf das zweite Griffstück beweglich ist. Insbesondere ist die erste Fingerauflage bei dieser Ausgestaltung der Erfindung nach Art einer Biegefeder, beispielsweise als elastisch biegbarer Federstab oder als eine Art Blattfeder ausgebildet. Die mit dem zweiten Griffstück einstückige und in der Betätigungsrichtung elastische Ausbildung der ersten Fingerauflage ermöglicht eine preisgünstige Herstellung des zweiten Griffstücks beispielsweise aus einem für chirurgische Instrumente geeigneten Metall durch beispielsweise Laserschneiden.

Eine erfindungsgemäße Weiterbildung sieht vor, dass die erste Fingerauflage als elastisch biegbare Federzunge, die an einem Ende einstückig in das zweite Griffstück übergeht, oder als elastisch biegbarer Federbügel, der an zwei Enden einstückig in das zweite Griffstück übergeht, ausgebildet ist. Die Federzunge weist ein freies Ende auf, das durch elastisches Biegen der Federzunge in der Betätigungsrichtung in die mit der zweiten Fingerauflage bündige Lage gebracht werden kann.

Eine Ausgestaltung der Erfindung sieht die Ausbildung der ersten Fingerauflage als elastisch in der Betätigungsrichtung biegbare Federzunge vor, die an einem Ende einstückig in das zweite Griffstück übergeht. Ein anderes Ende der Federzunge ist durch elastisches Biegen der Federzunge in der Bewegungsrichtung elastisch beweglich. Die zweite Fingerauflage ist bei dieser Ausgestaltung der Erfindung in Verlängerung der Federzunge und/oder seitlich neben dem beweglichen Ende der Federzunge angeordnet. In der Betätigungsrichtung gesehen weisen die beiden Fingerauflagen keinen Abstand voneinander oder einen kleinen Abstand voneinander auf derart, dass die beiden Fingerauflagen aneinander anliegen oder nur einen kleinen Spalt zwischen sich aufweisen, wenn sie bündig miteinander sind. In der Betätigungsrichtung gesehen kann die zweite Fingerauflage das bewegliche Ende der ersten Fingerauflage beispielsweise auch L-förmig oder U-förmig umschließen.

Eine Ausgestaltung der Erfindung sieht eine Seitenführung für die erste Fingerauflage vor, die die erste Fingerauflage in der Betätigungsrichtung beweglich an dem zweiten Griffstück führt. Die Seitenführung verhindert oder jedenfalls begrenzt eine Bewegung der ersten Fingerauflage quer zu der Betätigungsrichtung beispielsweise durch die Elastizität der ersten Fingerauflage.

Die beiden Fingerauflagen sind bei einer Ausgestaltung der Erfindung in einem Fingerring angeordnet. Der Fingerring ist ein Ring, der vorzugsweise starr an dem zweiten Griffstück angeordnet ist und zum Greifen des Griffstücks und zur Betätigung des chirurgischen Instruments mit einem Finger oder auch mit mehreren Fingern durchgegriffen werden kann. Insbesondere ist der Fingerring einstückiger Bestandteil des Griffstücks.

Es kann auch das erste Griffstück oder nur das erste Griffstück einen Fingerring aufweisen.

Eine Ausgestaltung der Erfindung sieht ein Griffstück mit zwei Fingerringen zum Greifen mit beispielsweise einem Zeigefinger und einem Mittelfinger, Ringfinger oder kleinen Finger der gleichen Hand vor. Möglich ist beispielsweise auch ein Fingerring an einem der Griffstücke für einen Daumen und einen oder mehrere weitere Fingerringe am gleichen Griffstück und/oder am anderen Griffstück für einen oder mehrere andere Finger der gleichen Hand.

Das erfindungsgemäße chirurgische Instrument ist insbesondere eine chirurgische Zange, Klemme, Schere oder dergleichen, es weist insbesondere einen Schaft mit zwei in einer Längsrichtung des Schafts gegeneinander verschieblichen Schaftteilen auf, die nebeneinander, vorzugsweise aneinander anliegend, oder ineinander, das heißt als Seele in einem Rohr als Hülle angeordnet sind. Mindestens eines der beiden Schaftteile ist mit einem der beiden Griffstücke antriebsverbunden derart, dass bei einer Bewegung der beiden Griffstücke zueinander die beiden Schaftteile gegeneinander verschoben werden. Das eine Schaftteil ist beispielsweise über ein Zwischenglied, das gelenkig mit dem einen Griffstück und gelenkig mit dem einen Schaftteil verbunden ist, gelenkig mit dem einen Griffstück verbunden. Das andere Griffstück kann ebenfalls gelenkig oder in anderer Weise mit dem anderen Schaftteil verbunden sein oder das andere Griffstück ist starr an dem anderen Schaftteil angeordnet.

An einem Ende weist der Schaft des chirurgischen Instruments beispielsweise Backen einer Klemme oder Zange oder Klingen oder eine Klinge und einen Amboss einer Schere auf. Die Backen, Klingen oder dergleichen werden durch die Verschiebung der beiden Schaftteile gegeneinander verschwenkt. Es können beide Backen, Klingen oder dergleichen schwenkbar sein oder eine Backe, Klinge oder dergleichen ist schwenkbar und die andere starr an dem Schaft des chirurgischen Instruments angeordnet.

Die beiden Griffstücke können pistolengriffartig quer oder schräg zu einer Seite von dem Schaft abstehen oder in Verlängerung des Schafts oder auch dem Schaft entgegen gerichtet an dem Schaft angeordnet sein.

Die Erfindung wird nachfolgend anhand eines in der Zeichnung dargestellten Ausführungsbeispiels näher erläutert. Es zeigen:
- Figur 1: ein chirurgisches Instrument gemäß der Erfindung;
- Figur 2: eine Vergrößerung einer Einzelheit des chirurgischen Instruments aus Figur 1 gemäß Pfeil II in Figur 1;
- Figur 3: die Einzelheit aus Figur 2 bei betätigtem chirurgischem Instrument; und
- Figuren 4 bis 8: erfindungsgemäße Abwandlungen der in Figur 2 gezeigten Einzelheit des chirurgischen Instruments aus Figur 1.

Grundsätzlich sind in den Figuren gleiche Teile mit gleichen Bezugszahlen versehen.

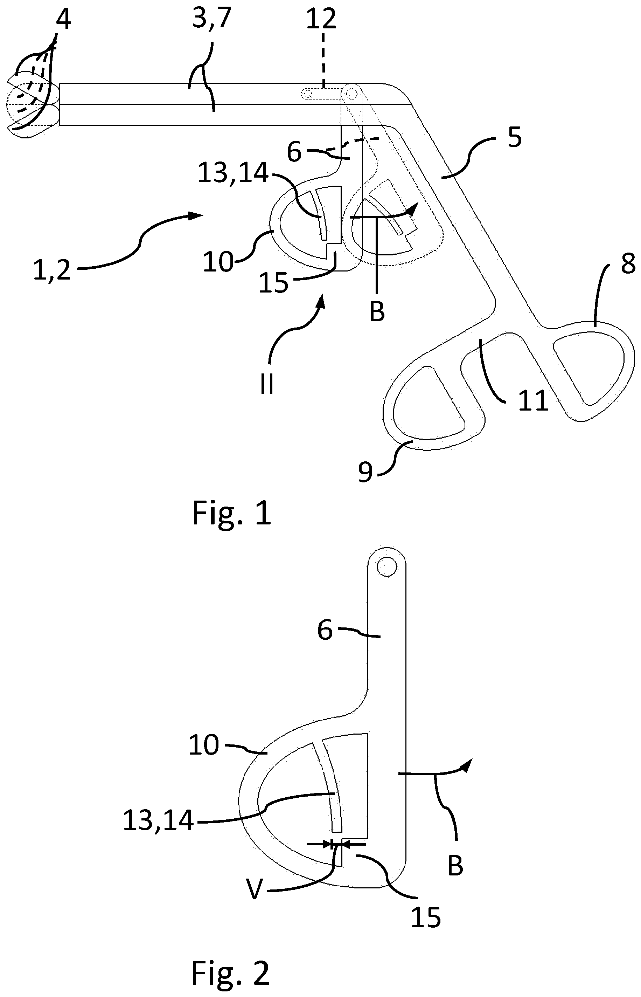

Als Ausführungsbeispiel eines erfindungsgemäßen chirurgischen Instruments 1 zeigt Figur 1 eine chirurgische Zange 2 mit einem Schaft 3, an dessen einem Ende zwei Backen 4 schwenkbar angeordnet und an dessen anderem Ende zwei Griffstücke 5, 6 angeordnet sind. Die als Ausführungsbeispiel der Erfindung gewählte Zange ist zum Implantieren einer nicht dargestellten Gehörknöchelchenprothese in ein Mittelohr eines Menschen vorgesehen. Andere Verwendungen der chirurgischen Zange 2 und andere Ausführungen des chirurgischen Instruments 1 beispielsweise als Schere sind gemäß der Erfindung möglich.

Der Schaft 3 ist gerade und stabförmig und weist zwei gerade, stabförmige Schaftteile 7 auf, die aneinander anliegend angeordnet und in einer Längsrichtung des Schafts 3 gegeneinander verschiebbar aneinander geführt sind. Durch eine Verschiebung der beiden Schaftteile 7 gegeneinander werden die beiden Backen 4 gegeneinander verschwenkt. Dazu brauchbare Schwenkmechaniken sind bekannt und werden hier nicht erläutert.

Die beiden Griffstücke 5, 6 sind - im Ausführungsbeispiel - ebenfalls gerade und stabförmig, wobei andere Formen möglich sind. Eines der beiden Griffstücke 5, das hier nachfolgend als erstes Griffstück 5 bezeichnet werden wird, ist starr an einem den Backen 4 fernen Ende eines der beiden Schaftteile 7 angeordnet. Im Ausführungsbeispiel steht das erste Griffstück 5 pistolengriffartig schräg zu einer Seite von dem Schaftteil 7 und damit auch vom Schaft 3 des erfindungsgemäßen chirurgischen Instruments 1 ab. Möglich ist beispielsweise auch ein senkrecht, das heißt radial von dem Schaft 3 abstehendes Griffstück 5 oder ein in axialer Verlängerung des Schafts 3 oder ein den Schaft 3 achsparallel oder schräg in einem Winkel verlängerndes Griffstück 5 (nicht dargestellt).

Das erste Griffstück 5 weist - im Ausführungsbeispiel - einen ersten Fingerring 8 an einem dem Schaft 3 fernen Ende auf. Außerdem - ebenfalls im Ausführungsbeispiel - weist das erste Griffstück 5 einen etwa rechtwinklig und nach vorn, das heißt in Richtung der Backen 4 von dem Griffstück 5 abstehenden Abzweig 11 auf, der an seinem Ende einen zweiten Fingerring 9 aufweist. Weder der Abzweig 11 noch die Fingerringe 8, 9 sind zwingend für die Erfindung. Im Ausführungsbeispiel ist der Abzweig 11 einstückig mit dem ersten Griffstück 5 und das erste Griffstück 5 einstückig mit dem einen Schaftteil 7.

Ein Ende eines zweiten der beiden Griffstücke 6 ist gelenkig mit dem anderen Schaftteil 7 verbunden, wobei das zweite Griffstück 6 - im Ausführungsbeispiel - in einer nicht betätigten Stellung, die Figur 1 mit durchgezogenen Linien zeigt, schräg nach vorn in einer gleichen Längsebene des Schafts 3 und zur gleichen Seite wie das erste Griffstück 5 von dem Schaft 3 des erfindungsgemäßen chirurgischen Instruments 1 absteht. Das zweite Griffstück 6 befindet sich vor dem ersten Griffstück 5, das heißt näher an den Backen 4 als das erste Griffstück 5 und das zweite Griffstück 6 lässt sich in der Längsebene des Schafts 3 in Richtung des ersten Griffstücks 5 schwenken. Allgemein ausgedrückt lassen sich die beiden Griffstücke 5, 6 aufeinander zu bewegen, das heißt ihr Abstand voneinander lässt sich verkleinern. Die Bewegung beziehungsweise das Schwenken des zweiten Griffstücks 6 in Richtung des ersten Griffstücks 5 ist eine Bewegung in einer Betätigungsrichtung B des chirurgischen Instruments 1. Mit Strichlinien zeigt Figur 1 das zweite Griffstück 6 in einer betätigten Stellung, das heißt in der Betätigungsrichtung in Richtung des ersten Griffstücks 5 bewegt beziehungsweise geschwenkt.

Im Ausführungsbeispiel weist auch das zweite Griffstück 6 einen Fingerring 10 auf, der hier nachfolgend auch als dritter Fingerring 10 bezeichnet werden wird. Wie oben geschrieben sind die Fingerringe 8, 9 ,10 nicht zwingend für die Erfindung. Die Fingerringe 8, 9, 10 dienen einem Halten und Betätigen des chirurgischen Instruments 1: In beziehungsweise durch die Fingerringe 8, 9, 10 lassen sich Finger einer Hand einer das chirurgische Instrument 1 benutzenden Person stecken. Zum Halten des chirurgischen Instruments 1 wird beispielsweise ein Zeigefinger durch oder in den dritten Fingerring 10 an dem dem Schaft 3 fernen Ende des zweiten Griffstücks 6, ein Mittelfinger derselben Hand durch oder in den zweiten Fingerring 9 am Ende des Abzweigs 11 des ersten Griffstücks 5 und ein Daumen derselben Hand durch oder in den ersten Fingerring 8 an dem dem Schaft 3 fernen Ende des ersten Griffstücks 5 gesteckt (nicht dargestellt). Zum Betätigen des chirurgischen Instruments 1 wird das zweite Griffstück 6 in der Betätigungsrichtung B, das heißt in Richtung des ersten Griffstücks 5 geschwenkt. Allgemein ausgedrückt werden die beiden Griffstücke 5, 6 zum Betätigen des chirurgischen Instruments 1 einander genähert. Möglich sind auch erfindungsgemäße chirurgische Instrumente 1, die durch Auseinanderschwenken beziehungsweise voneinander Entfernen ihrer Griffstücke 5, 6 betätigt werden (nicht dargestellt).

Das schwenkbare, zweite Griffstück 6 ist nahe an seiner schwenkbaren Lagerung an dem einen Schaftteil 7 gelenkig mit einem Zwischenglied 12 verbunden, das seinerseits gelenkig mit dem anderen Schaftteil 7 verbunden ist derart, dass durch Schwenken oder allgemein Bewegen des zweiten Griffstücks 6 das andere Schaftteil 7 gegenüber dem einen Schaftteil 7 in der Längsrichtung des Schafts 3 verschoben wird. Bei einer Bewegung des zweiten Griffstücks 6 in der Betätigungsrichtung B werden dadurch die beiden Backen 4 aufeinander zu und bei Bewegung in einer entgegengesetzten Löserichtung die beiden Backen 4 auseinander geschwenkt.

Das erfindungsgemäße chirurgische Instrument 1 weist erste Fingerauflage 13 auf einer dem ersten Griffstück 5 abgewandten Seite des zweiten Griffstücks 6 auf (Figur 2). Im Ausführungsbeispiel befindet sich die erste Fingerauflage 13 in dem dritten Fingerring 10 am Ende des zweiten Griffstücks 6. Die erste Fingerauflage 13 ist in der Betätigungsrichtung B, das heißt in Richtung des ersten Griffstücks 5 elastisch beweglich an dem zweiten Griffstück 6. Im Ausführungsbeispiel weist die erste Fingerauflage 13 eine streifenförmige Federzunge 14 auf, die auf der dem ersten Griffstück 5 abgewandten Seite des zweiten Griffstücks 6 in einem geringen Abstand von etwa einem oder wenigen Millimetern von dem zweiten Griffstück 6 und parallel oder in einem spitzen Winkel zu dem zweiten Griffstück 6 angeordnet ist (Figur 2). Ein Ende der Federzunge 14 geht einstückig in das zweite Griffstück 6 über, im Ausführungsbeispiel geht das Ende der Federzunge 14 nahe dem zweiten Griffstück 6 einstückig in den dritten Fingerring 10 über, in dem die Federzunge 14 im Ausführungsbeispiel angeordnet ist. Ein anderes, freies Ende der die erste Fingerauflage 13 bildenden Federzunge 14 ist durch elastisches Biegen der Federzunge 14 in Richtung des zweiten Griffstücks 6 und damit in der Bewegungsrichtung beweglich.

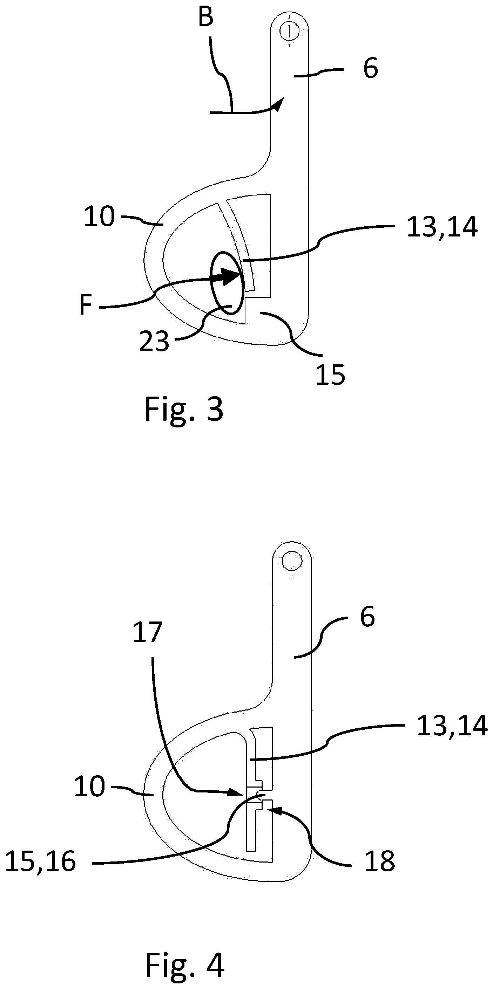

Zur Betätigung des erfindungsgemäßen chirurgischen Instruments 1 wird eine Betätigungskraft F (Figur 3) in der Betätigungsrichtung B (Figuren 1 bis 3) auf die erste Fingerauflage 13 ausgeübt, die die erste Fingerauflage 13 elastisch in Richtung des zweiten Griffstücks 6 biegt beziehungsweise verformt, wobei die erste Fingerauflage 13 die Betätigungskraft F auf das zweite Griffstück 6 überträgt, wodurch das zweite Griffstück 6 in der Betätigungsrichtung B geschwenkt beziehungsweise bewegt und das chirurgische Instrument 1 in oben beschriebener Weise betätigt wird.

In Verlängerung der die erste Fingerauflage 13 bildenden Federzunge 14 weist das zweite Griffstück 6 eine zweite Fingerauflage 15 auf, die starr und ebenfalls auf der dem ersten Griffstück 5 abgewandten Seite des zweiten Griffstücks 6 angeordnet ist. Im Ausführungsbeispiel befindet sich die zweite Fingerauflage 15 ebenfalls im dritten Fingerring 10. Im Ausführungsbeispiel ist die zweite Fingerauflage 15 als Stufe auf der dem ersten Griffstück 5 abgewandten Seite des zweiten Griffstücks 6 ausgebildet, die sich im Ausführungsbeispiel im dritten Fingerring 10 befindet.

Die zweite Fingerauflage 15 weist einen Versatz V in der Betätigungsrichtung B, das heißt im Ausführungsbeispiel in Richtung des ersten Griffstücks 5 in Bezug auf die erste Fingerauflage 13 beziehungsweise in Bezug auf die Federzunge 14 beziehungsweise das freie Ende der Federzunge 14 auf, wenn die Federzunge 14 unbelastet und infolge dessen unverformt ist. Der Versatz V beträgt beispielsweise etwa 1 mm.

Wird die Betätigungskraft F in der Betätigungsrichtung B auf die erste Fingerauflage 13 ausgeübt, lässt sich die erste Fingerauflage 13 elastisch so weit verformen, bis sie bündig mit der zweiten Fingerauflage 15 ist, das heißt das freie Ende der Federzunge 14 ist bündig mit der zweiten Fingerauflage 15. Die erste Fingerauflage 13 lässt sich auch über die bündige Lage mit der zweiten Fingerauflage 15 hinweg in Richtung des ersten Griffstücks 6 elastisch verformen.

In der Betätigungsrichtung B gesehen weisen die beiden Fingerauflagen 13, 15 keinen Abstand oder einen kleinen Abstand von vorzugsweise einigen Zehntelmillimeter voneinander auf derart, dass kein oder nur ein kleiner Spalt zwischen den beiden Fingerauflagen 13, 15 besteht, wenn die erste Fingerauflage 13 elastisch in die mit der zweiten Fingerauflage 15 bündige Lage verformt ist.

Betätigt wird das erfindungsgemäße chirurgische Instrument 1mit einem Finger 23 (Figur 3), beispielsweise einem Zeigefinger, der auf der dem ersten Griffstück 5 abgewandten Seite auf die erste Fingerauflage 13 gelegt und mit der Betätigungskraft F in der Betätigungsrichtung B, das heißt in Richtung des ersten Griffstücks 5 gegen die erste Fingerauflage 13 drückt. Die auf die erste Fingerauflage 13 ausgeübte Betätigungskraft F verformt - im Ausführungsbeispiel biegt - die erste Fingerauflage 13 elastisch in der Betätigungsrichtung B, das heißt in Richtung des zweiten Griffstücks 6. Wird die erste Fingerauflage 13 so weit verformt, elastisch bewegt beziehungsweise gebogen, bis ihr freies Ende bündig mit der zweiten Fingerauflage 13 ist, stößt der Finger 23, der gegen die erste Fingerauflage 13 drückt, an oder gegen die zweite - im Ausführungsbeispiel starre - Fingerauflage 15, wie es Figur 3 zeigt. Das Stoßen des Fingers 23 gegen beziehungsweise das Anstoßen des Fingers 23 an die zweite Fingerauflage 15 bei der Betätigung des chirurgischen Instruments 1 wird haptisch als eine Art "Druckpunkt" wahrgenommen. Eine das chirurgische Instrument 1 mit dem Finger 23 benutzende Person spürt diesen Druckpunkt bei der Betätigung des erfindungsgemäßen chirurgischen Instruments 1.

Dem Druckpunkt beziehungsweise der bündigen Lage der beiden Fingerauflagen 13, 15 ist eine bestimmte Betätigungskraft F zugeordnet, weil sie eine elastische Biegung beziehungsweise Verformung der die erste Fingerauflage 13 bildenden Federzunge 14 erfordert, die dem Versatz V der beiden Fingerauflagen 13, 15 in der Betätigungsrichtung B entspricht, wenn die Federzunge 14 bei nicht betätigtem chirurgisches Instrument 1 unverformt ist. Die elastische Verformung beziehungsweise Biegung der die erste Fingerauflage 13 bildenden Federzunge 14 bis zu dem Druckpunkt, das heißt bis zu der mit der zweiten Fingerauflage 15 bündigen Lage oder Position erfordert eine entsprechende Biegekraft und damit die bestimmte Betätigungskraft, die dem Druckpunkt des chirurgischen Instruments 1 zugeordnet ist. Die Betätigung des chirurgischen Instruments 1 kann bei Erreichen des haptisch wahrnehmbaren Druckpunkts beendet werden, wodurch die Betätigungskraft F auf die dem Druckpunkt zugeordnete Betätigungskraft F begrenzt wird. Es kann auch - an sich umgekehrt - das chirurgisches Instrument 1 bis (mindestens) zum Druckpunkt betätigt werden, um ein Zusammenklemmen der beiden Backen 4 mit (mindestens) einer dieser Betätigungskraft F zugeordneten Klemmkraft und damit ein zuverlässiges Festklemmen beispielsweise einer nicht dargestellten Gehörknöchelchenprothese oder eines anderen Gegenstands zwischen den Backen 4 des - im Ausführungsbeispiel als chirurgische Zange 2 ausgebildeten - chirurgischen Instruments 1 sicher zu stellen.

Die Federkraft und damit die Betätigungskraft F am "Druckpunkt" bei bündigen Fingerauflagen 13, 15 lässt sich durch Form und Anordnung der die erste Fingerauflage 13 bildenden Federzunge 14, insbesondere durch eine Dicke der Federzunge 14 festlegen.

Figur 3 zeigt eine erfindungsgemäße Abwandlung der die erste Fingerauflage 13 bildenden Federzunge 14 und der zweiten Fingerauflage 15: wie in Figuren 1 und 2 befindet sich die die erste Fingerauflage 13 bildende Federzunge 14 in Figur 3 ebenfalls auf der dem ersten Griffstück 5 abgewandten Seite des zweiten Griffstücks 6 in dem dritten Fingerring 10 und geht an einem Ende einstückig in das zweite Griffstück 6 beziehungsweise in den dritten Fingerring 10 über, der einstückig mit dem zweiten Griffstück 6 ist.

Im Unterschied zu Figuren 1 bis 3 ist in Figur 4 die zweite Fingerauflage 15 nicht am freien Ende der die erste Fingerauflage 13 bildenden Federzunge 14 angeordnet, sondern die zweite Fingerauflage 15 ist in Figur 4 als Stift 16 ausgebildet, der in einem Mittelbereich zwischen den beiden Enden der Federzunge 14 angeordnet ist und der von der dem ersten Griffstück 5 abgewandten Seite des zweiten Griffstücks 6 in Richtung der Federzunge 14 absteht. Im Ausführungsbeispiel ist der die zweite Fingerauflage 15 bildende Stift 16 einstückig mit dem zweiten Griffstück 6.

Der die zweite Fingerauflage 15 bildende Stift 16 ragt in ein Loch als Öffnung 17 in der die erste Fingerauflage 13 bildenden Federzunge 14, wobei der Stift 16 nicht bis zu der dem ersten Griffstück 5 abgewandten Seite oder Fläche der Federzunge 14 reicht, wenn die Federzunge 14 unverformt ist. Wird die Federzunge 14 zur Betätigung des erfindungsgemäßen chirurgischen Instruments 1 elastisch in Richtung des zweiten Griffstücks 6 gebogen, wird die dem zweiten Griffstück 6 abgewandte Seite oder Fläche der Federzunge 14 bündig mit einem dem zweiten Griffstück 6 fernen Stirnende des Stifts 16, der die zweite Fingerauflage 15 bildet. Die mit der zweiten Fingerauflage 15 bündige Lage der die erste Fingerauflage 13 bildenden Federzunge 14 ist der oben zu Figuren 1 und 2 erläuterte "Druckpunkt", der wie erläutert bei der der Betätigung des chirurgischen Instruments 1 haptisch mit dem Finger wahrgenommen wird und dem die bestimmte Betätigungskraft F zugeordnet ist.

Der von dem zweiten Griffstück 6 abstehenden Stift 16 und das die Öffnung 17 bildende Loch in der Federzunge 14 bilden eine Seitenführung 18, die die die erste Fingerauflage 13 bildende Federzunge 14 in der Betätigungsrichtung B führt und gegen eine Bewegung quer zur Betätigungsrichtung B abstützt.

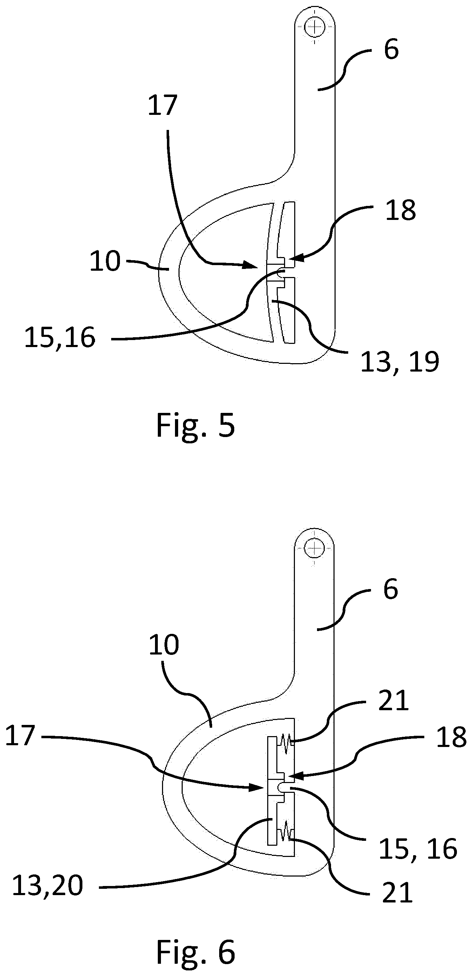

Im Unterschied zur vorstehend erläuterten Figur 5 ist die erste Fingerauflage 13 in der in Figur 5 dargestellten erfindungsgemäßen Abwandlung des chirurgischen Instruments 1 nicht als an einem Ende freie Federzunge ausgebildet, sondern als - im Ausführungsbeispiel gewölbter - Federbügel 19, der an beiden Enden einstückig in das zweite Griffstück 6 beziehungsweise in den dritten Fingerring 10 übergeht. Im Übrigen sind in Figur 5 die Fingerauflagen 13, 15 gleich ausgebildet wie in Figur 4 und es werden die Ausführungen zu Figur 4 zur Erläuterung der Figur 5 in Bezug genommen.

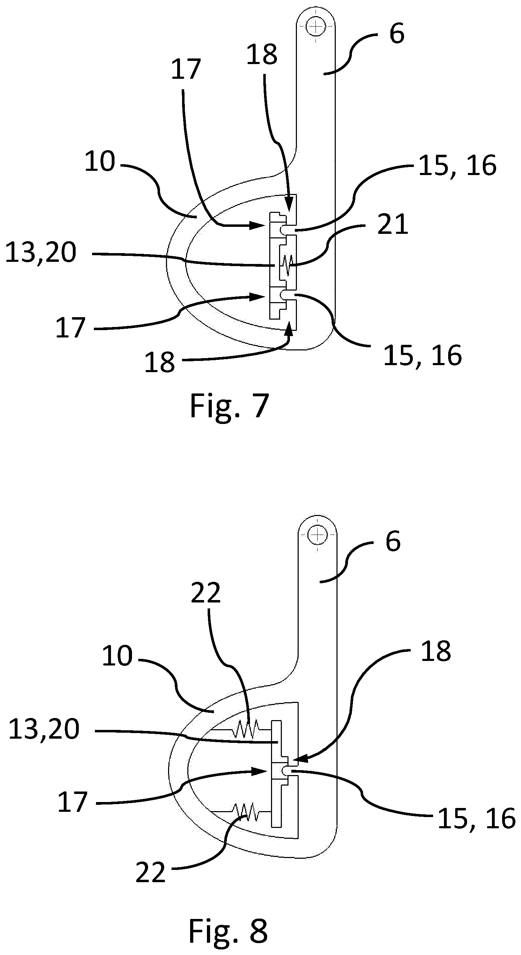

Die in Figuren 6, 7 und 8 dargestellten, erfindungsgemäßen Abwandlungen der Fingerauflagen 13, 15 weisen eine streifenförmige Platte 20 als erste Fingerauflage 13 auf, die auf der dem ersten Griffstück 5 abgewandten Seite des zweiten Griffstücks 6 - in den Ausführungsbeispielen in dem dritten Fingerring 10 - angeordnet ist. Die Platte 20 ist wie die Federzunge 14 in Figuren 1 bis 3 und 4 und wie der Federbügel 19 in Figuren 6 und 8 mit einem Stift 16 und in Figur 7 mit zwei Stiften 16, die von der dem ersten Griffstück 5 abgewandten Seite des zweiten Griffstücks 6 abstehen und als Seitenführungen 18 jeweils in ein Loch als Öffnung 17 in der Platte 20 greifen in der Betätigungsrichtung B des zweiten Griffstücks 6 an dem zweiten Griffstück 6 beweglich geführt.

In Figur 6 stützen zwei Schraubendruckfedern 21 und in Figur 7 eine Schraubendruckfeder 22 die Platte 20, die die erste Fingerauflage 13 bildet, elastisch an dem zweiten Griffstück 6 ab und in Figur 7 halten zwei Schraubenzugfedern 22 die Platte 20 in Richtung des zweiten Griffstücks 6 elastisch am dritten Fingerring 10.

Wie zu Figur 3 erläutert reicht der die zweite Fingerauflage 15 bildende Stift 16 nicht bis zu der dem ersten Griffstück 5 abgewandten Seite oder Fläche der die erste Fingerauflage 13 bildenden Platte 20, wenn die Federn 21, 22 unverformt sind. Wird die Platte 20 zur Betätigung des erfindungsgemäßen chirurgischen Instruments 1 gegen die Federkraft der Federn 21, 22 in der Betätigungsrichtung B zum zweiten Griffstück 6 gedrückt, gelangt die Platte 20 in die mit dem Stirnende des Stifts 16 bündige Lage, was als Druckpunkt mit dem Finger 23 spürbar ist, der die Platte 20 mit der Betätigungskraft F in der Betätigungsrichtung B drückt.

Ergänzend werden zur Erläuterung der Figuren 5 bis 8 die Erläuterungen der Figu-ren 1 bis 4 in Bezug genommen.

Die Federzunge 14, der Federbügel 19, die Schraubendruckfeder 21 und die Schraubenzugfeder 22 können allgemein auch als Federelemente aufgefasst werden, die die erste Fingerauflage 13 in Richtung des ersten Griffstücks 5 elastisch beweglich am zweiten Griffstück 6 halten.

## Patentansprüche

1. Chirurgisches Instrument, mit einem ersten Griffstück (5) und mit einem zweiten Griffstück (6), das zu einer Betätigung des chirurgischen Instruments (1) in Bezug auf das erste Griffstück (5) beweglich ist, wobei das zweite Griffstück (6) eine erste Fingerauflage (13) auf einer dem ersten Griffstück (5) abgewandten Seite aufweist, die in Bezug auf das zweite Griffstück (6) elastisch in Richtung des ersten Griffstücks (5) beweglich ist, **dadurch gekennzeichnet, dass** das zweite Griffstück (6) eine zweite Fingerauflage (15) auf seiner dem ersten Griffstück (5) abgewandten Seite aufweist, die bei unbelasteter erster Fingerauflage (13) in Bezug auf die erste Fingerauflage (13) in Richtung des ersten Griffstücks (5) versetzt ist und mit der die erste Fingerauflage (13) bündig wird oder ist, wenn die erste Fingerauflage (13) mit einer Betätigungskraft (F) in Richtung des ersten Griffstücks (5) beaufschlagt und dadurch elastisch in Bezug zu dem zweiten Griffstück (6) bewegt wird oder ist.

2. Chirurgisches Instrument nach Anspruch 1, **dadurch gekennzeichnet, dass** das chirurgische Instrument (1) ein Federelement (14; 19; 21; 22) aufweist, das die erste Fingerauflage (13) in Richtung des ersten Griffstücks (5) elastisch beweglich am zweiten Griffstück (6) hält.

3. Chirurgisches Instrument nach Anspruch 1, **dadurch gekennzeichnet, dass** die erste Fingerauflage (13) einstückig mit dem zweiten Griffstück (6) und elastisch in Richtung des ersten Griffstücks (5) verformbar ist.

4. Chirurgisches Instrument nach Anspruch 3, **dadurch gekennzeichnet, dass** die erste Fingerauflage (13) eine elastisch biegbare Federzunge (14), die an einem Ende einstückig in das zweite Griffstück (6) übergeht, oder einen Federbügel (19), der an zwei Enden einstückig in das zweiten Griffstück (6) übergeht, aufweist.

5. Chirurgisches Instrument nach Anspruch 4, **dadurch gekennzeichnet, dass** die erste Fingerauflage (13) die elastisch biegbare Federzunge (14) aufweist und dass die zweite Fingerauflage (15) in Verlängerung der Federzunge (14) an einem beweglichen Ende der Federzunge (14) an dem zweiten Griffstück (6) angeordnet ist derart, dass die Federzunge (14) elastisch in Richtung des ersten Griffstücks (5) in eine mit der zweiten Fingerauflage (15) bündige Lage gebogen werden kann.

6. Chirurgisches Instrument nach einem oder mehreren der vorhergehenden Ansprüche, **dadurch gekennzeichnet, dass** die zweite Fingerauflage (15) in einer Öffnung (17), an einem Rand oder an einem Ende der ersten Fingerauflage (13) angeordnet ist.

7. Chirurgisches Instrument nach einem oder mehreren der vorhergehenden Ansprüche, **dadurch gekennzeichnet, dass** das zweite Griffstück (6) eine Seitenführung (18) für die erste Fingerauflage (13) aufweist, die die erste Fingerauflage (13) in der Bewegungsrichtung des zweiten Griffstücks (6) in Bezug zum ersten Griffstück (5) führt.

8. Chirurgisches Instrument nach einem oder mehreren der vorhergehenden Ansprüche, **dadurch gekennzeichnet, dass** die Fingerauflagen (13, 15) in einem Fingerring (10) angeordnet sind.

9. Chirurgisches Instrument nach einem oder mehreren der vorhergehenden Ansprüche, **dadurch gekennzeichnet, dass** ein Griffstück (5) zwei Fingerringe (8, 9) aufweist.

## Claims

1. Surgical instrument, comprising a first handle piece (5) and comprising a second handle piece (6) which is movable with respect to the first handle piece (5) in order to actuate the surgical instrument (1), the second handle piece (6) having a first finger rest (13) on a side remote from the first handle piece (5), which first finger rest is elastically movable with respect to the second handle piece (6) in the direction of the first handle piece (5), **characterized in that** the second handle piece (6) has a second finger rest (15) on its side remote from the first handle piece (5), which second finger rest, when the first finger rest (13) is not subject to load, is offset with respect to the first finger rest (13) in the direction of the first handle piece (5) and with which second finger rest the first finger rest (13) becomes or is flush when the first finger rest (13) is subjected to an actuating force (F) in the direction of the first handle piece (5) and thereby moves or is moved elastically with respect to the second handle piece (6).

2. Surgical instrument according to claim 1, **characterized in that** the surgical instrument (1) comprises a spring element (14; 19; 21; 22) which holds the first finger rest (13) on the second handle piece (6) so as to be elastically movable in the direction of the first handle piece (5).

3. Surgical instrument according to claim 1, **characterized in that** the first finger rest (13) is integral with the second handle piece (6) and is elastically deformable in the direction of the first handle piece (5).

4. Surgical instrument according to claim 3, **characterized in that** the first finger rest (13) comprises an elastically bendable spring tongue (14) which merges integrally into the second handle piece (6) at one end, or a spring clip (19) which merges integrally into the second handle piece (6) at two ends.

5. Surgical instrument according to claim 4, **characterized in that** the first finger rest (13) comprises the elastically bendable spring tongue (14) and **in that** the second finger rest (15) is arranged in the extension of the spring tongue (14) at a movable end of the spring tongue (14) on the second handle piece (6) in such a way that the spring tongue (14) can be bent elastically in the direction of the first handle piece (5) into a position flush with the second finger rest (15).

6. Surgical instrument according to one or more of the preceding claims,
**characterized in that** the second finger rest (15) is arranged in an opening (17), at an edge or at an end of the first finger rest (13).

7. Surgical instrument according to one or more of the preceding claims,
**characterized in that** the second handle piece (6) comprises a lateral guide (18) for the first finger rest (13), which guides the first finger rest (13) in the direction of movement of the second handle piece (6) with respect to the first handle piece (5).

8. Surgical instrument according to one or more of the preceding claims,
**characterized in that** the finger rests (13, 15) are arranged in a finger ring (10).

9. Surgical instrument according to one or more of the preceding claims, **characterized in that** a handle piece (5) comprises two finger rings (8, 9).

## Revendications

1. Instrument chirurgical, comportant une première pièce de préhension (5) et comportant une seconde pièce de préhension (6) pouvant être déplacée par rapport à la première pièce de préhension (5) pour un actionnement de l'instrument chirurgical (1), dans lequel la seconde pièce de préhension (6) présente un premier support pour doigts (13) sur un côté opposé à la première pièce de préhension (5), lequel premier support pour doigts peut être déplacé de manière élastique par rapport à la seconde pièce de préhension (6) en direction de la première pièce de préhension (5), **caractérisé en ce que** la seconde pièce de préhension (6) présente un second support pour doigts (15) sur son côté opposé à la première pièce de préhension (5), lequel second support pour doigts est décalé par rapport au premier support pour doigts (13) en direction de la première pièce de préhension (5) lorsque le premier support pour doigts (13) n'est pas chargé, et avec lequel le premier support pour doigts (13) est en affleurement, lorsque le premier support pour doigts (13) est soumis à une force d'actionnement (F) en direction de la première pièce de préhension (5) et est ainsi déplacé de manière élastique par rapport à la seconde pièce de préhension (6).

2. Instrument chirurgical selon la revendication 1, **caractérisé en ce que** l'instrument chirurgical (1) présente un élément élastique (14 ; 19 ; 21 ; 22) qui maintient le premier support pour doigts (13) sur la seconde pièce de préhension (6) de manière à pouvoir le déplacer élastiquement en direction de la première pièce de préhension (5).

3. Instrument chirurgical selon la revendication 1, **caractérisé en ce que** le premier support pour doigts (13) est solidaire de la seconde pièce de préhension (6) et est élastiquement déformable en direction de la première pièce de préhension (5).

4. Instrument chirurgical selon la revendication 3, **caractérisé en ce que** le premier support pour doigts (13) présente une languette élastique (14) pouvant être pliée élastiquement qui, à une extrémité, se prolonge de manière solidaire dans la seconde pièce de préhension (6), ou un arceau élastique (19) qui, à deux extrémités, se prolonge de manière solidaire dans la seconde pièce de préhension (6).

5. Instrument chirurgical selon la revendication 4, **caractérisé en ce que** le premier support pour doigts (13) présente la languette élastique (14) pouvant être pliée élastiquement **et en ce que** le second support pour doigts (15) est disposé dans un prolongement de la languette élastique (14), au niveau d'une extrémité pouvant être déplacée de la languette élastique (14) sur la seconde pièce de préhension (6), de telle sorte que la languette élastique (14) peut être pliée élastiquement en direction de la première pièce de préhension (5) dans une position en affleurement avec le second support pour doigts (15).

6. Instrument chirurgical selon l'une ou plusieurs des revendications précédentes, **caractérisé en ce que** le second support pour doigts (15) est disposé dans une ouverture (17), sur un bord ou au niveau d'une extrémité du premier support pour doigts (13).

7. Instrument chirurgical selon l'une ou plusieurs des revendications précédentes, **caractérisé en ce que** la seconde pièce de préhension (6) présente un guide latéral (18) pour le premier support pour doigts (13), lequel guide latéral guide le premier support pour doigts (13) dans la direction de déplacement de la seconde pièce de préhension (6) par rapport à la première pièce de préhension (5).

8. Instrument chirurgical selon l'une ou plusieurs des revendications précédentes, **caractérisé en ce que** les supports pour doigts (13, 15) sont disposés dans un anneau pour doigts (10).

9. Instrument chirurgical selon l'une ou plusieurs des revendications précédentes, **caractérisé en ce qu'**une pièce de préhension (5) présente deux anneaux pour doigts (8, 9).
